# EUROPEAN PATENT APPLICATION

(11) **EP 2 409 686 A1**
(43) Date of publication of application: **25.01.2012**
(21) Application number: 11180288.0
(22) Date of filing: 01.08.2003
(51) Int. Cl.: A61K 9/12, A61K 9/14, A61K 47/48, A61K 9/16, A61K 9/00

(54) **Cell transport compositions and uses thereof**

(30) Priority: 01.08.2002 US 400159 P; 28.08.2002 US 406525 P; 18.11.2002 US 427388 P; 22.07.2003 US 489191 P
(62) Divisional of application: 03767137.7
(71) Applicant: Mannkind Corporation, Valencia, California 91355 (US)
(72) Inventor: Gelber, Cohava, Hartsdale, NY New York 10530 (US); Rousseau, Kathleen, Pleasant Valley, NY New York 12569 (US)
(74) Representative: Baker, Colin John

(57) **Abstract**

A method for transporting a compound across a membrane or lipid bilayer, comprising contacting a proximal face of the membrane or bilayer with a complex comprising the compound and diketopiperazine (DKP), wherein transport of the compound from the proximal face of the lipid bilayer to a distal face of the lipid bilayer is increased in the presence of the DKP compared to in the absence of the DKP.

## Description

### BACKGROUND OF THE INVENTION

The invention relates to drug delivery compositions and methods of use thereof.

Many therapeutic compounds are not clinically useful, because they fall victim to a solubility paradox, which makes them unsuited for commercial development. The compounds can travel through an aqueous environment to reach target cells, but then cannot reach an intracellular target, because of the difficulties in crossing the non-polar lipid bilayer of a cell. Standard means of drug administration are limited in their efficiency and their ability to target certain tissues. Moreover, some drug delivery agents produce undesirable side effects, such as inflammation and toxicity.

It is therefore an object of the present invention to provide methods and compositions for transporting compounds across membranes with little or no toxicity.

### SUMMARY OF THE INVENTION

Compositions and methods have been developed for transporting compounds across membranes with little or no toxicity and, when targeted through the appropriate routes of administration (i.e., lung, gastrointestinal (GI) tract), little or no immune stimulation. The compositions can mediate cellular delivery of compounds that would otherwise not enter cells and enhance the intracellular delivery of compounds that would otherwise enter cells inefficiently.

The methods for transporting a composition across a lipid bilayer are carried out by contacting a proximal face of a lipid bilayer (e.g. the surface of an intact cell) with a complex containing a compound (e.g., a therapeutic agent) and a diketopiperazine (DKP). DKP and the compound are non-covalently associated with each other or covalently bound to each other. Compared to the rate of transport for compounds that are not complexed with DKP, the rate of transport from the proximal face of the lipid bilayer (e.g., an extracellular membrane face) to a distal face of the lipid bilayer (e.g., intracellular membrane face or cytoplasm of the cell) for compositions containing compounds that are complexed with DKP is greater due to the presence of the DKP.

### BRIEF DESCRIPTION OF THE DRAWINGS

Figure 1a is a line graph of mcg/ml or % versus stimulated index, showing a mitogenic response of naive spleen cells to Fumaryl DKP (FDKP)-microspheres (TECHNOSPHERE^{®}).
Figure 1b is a bar showing a cytokine analysis of supernatant from an *in vitro* mitogenicity study of naive spleen cells in the presence of clinical grade TECHNOSPHERE^{®}.
Figure 2a is a bar graph showing an *in vitro* mitogenicity study of human PBMC's in the presence of varying batches of clinical grade or crude TECHNOSPHERE^{®}.
Figure 2b is a bar graph showing a cytokine analysis of supernatant from an *in vitro* mitogenicity study of the PBMCs in the presence TECHNOSPHERE^{®} batches of naïve spleen cells.
Figure 3a is a bar graph of time (minutes) versus mean fluoresence intensity (MFI) (units) showing the kinetics of ovalbumin (OVA) -FITC transport into an A459 human lung cell line following incubation with a 20 micrograms/ml preparation of either OVA-FITC or OVA-FITC-FTS FDKP ("OVA*TECH-FITC") at 37°C.
Figure 3b is a bar graph of time (minutes) versus MFI depicting the transport enhancement (expressed in %) of OVA-FITC into A459 cells incubated with a 20 micrograms/ml preparation of either OVA-FITC or OVA-FITC-FDKP ("OVA*TECH-FITC") at 37°C.
Figure 4 is a bar graph incubation temperature (°C) versus MFI (Units) showing enhancement of transport of ovalbumin by FDKP-microspheres into A459 human lung cells after a 30-minute incubation with 20 micrograms/ml of either OVA-FITC-succinyl or OVA-FITC-FDKP (OVA*TECH-FITC) at 37°C, 4°C, and 0°C.
Figure 5 is a bar graph of time (minutes) versus MFI (Units) showing FDKP-microsphere-facilitated transport of ovalbumin in uncultured spleen cells at 37°C.
Figure 6 is a bar graph showing transport of ovalbumin into A459 lung cells in the presence of complete medium.
Figure 7 is a bar graph showing transport of ovalbumin into A459 lung cells in the presence of phenylarsine oxide.
Figure 8 is a bar graph showing transport of ovalbumin into A459 lung cells in the presence of sucrose.
Figure 9 is a bar graph depicting the transport of FITC-OVA into K562 cells following incubation with a 20 micrograms/ml preparation of either OVA-FITC or OVA-FITC-FDKP ('OVA*TECH-FITC') at 37°C and various pH conditions (3, 4, 5, 7.4 and 9).
Figure 10 is a line graph of time (minutes) versus MFI (Units) comparing transport of insulin to trasnport of insulin/FDKP into A459 lung cells at 37° C.
Figure 11 is a bar graph showing insulin-specific IgG antibody titers in human subjects before ("baseline") and after ("endpoint") administration of insulin/FDKP-microsphere complexes by inhalation therapy.

### DETAILED DESCRIPTION

The compositions and methods described herein improve the transport of compounds through a membrane by complexing the compound with DKP. DKP improves the therapeutic performance of molecules through efficient delivery to target cells and tissues and thus allow for treatment with a lower dose. Optionally, DKP is coated with a synthetic or natural polymer.

As generally used herein "substantially no immune response" means that the immune response is increased by less than 50% in the presence of the DKP compared to in its absence. Preferably, the immune response increases less than 20%, less than 10%, less than 5%, or not at all. An immune response is measured by detecting antibody production, cytokine secretion (e.g., interleukin-2), or proliferation of immune cells such as T cells. The DKP or complex bind to receptors, which participate in induction of innate immunity such as those that recognize pathogen-associated molecular patterns. For example, the DKP or compound-DKP complex does not engage a toll-like receptor 2.

### I. Compositions

### A. Compounds

A variety of different compounds can be complexed with FDKP for delivery to target cells, such as lung alveolar cells. The compounds may be peptides or proteins, oligo or polysaccharides, nucleic acid molecules, and combinations of these compounds. Compounds to be delivered include synthetic molecules, synthetic small molecules or molecules such as metals. The compositions are conjugated to or complexed with a DKP.

Compounds to be transported include biologically active agents. Compounds to be delivered include large proteins, polypeptides, nucleic acids, carbohydrates, and small molecules. Preferably, the compound is a polypeptide. To minimize immune responsiveness, the amino acid sequence of the polypeptide is identical or homologous to a naturally occurring polypeptide expressed by a member of the species of the mammal to which the composition is delivered. For example, the compound can be a peptide such as insulin or a biologically active fragment thereof, Parathyroid hormone (PTH), Calcitonin, Human Growth Hormone (HgH), Glucagon-like peptides (GLP), or a fragment thereof. The compound can also be an antibodys or antigen-binding fragment thereof, e.g., an antibody that binds to a pathogenic infectious agent, malignant cell, or pathogenic molecule. The antibody can be an intact monoclonal antibody or an immunologically-active antibody fragment, e. g., a Fab or (Fab)₂ fragment; an engineered single chain Fv molecule; or a chimeric molecule, e.g., an antibody which contains the binding specificity of one antibody, e.g., of murine origin, and the remaining portions of another antibody, e.g., of human origin.

The compound may be a cytokine or chemokine. Chemokines are a superfamily of small proteins, which play an important role in recruiting inflammatory cells into tissues in response to infection and inflammation. Chemokines facilitate leukocyte migration and positioning as well as other processes such as angiogenesis and leukocyte degranulation. Cytokines act as messengers to help regulate immune and inflammatory responses. When in suboptimal concentration, a proper immune response fails to be evoked. In excess, cytokines can be harmful and have been linked to a variety of diseases. Addition of blocking cytokines and growth factors in accordance with the treatment goal, is a proven therapeutic approach with a number of drugs already approved or in clinical development.

The cytokine superfamily includes factors such as erythropoietin, thrombopoietin, granulocyte-colony-stimulating factor (GCSF) and the interleukins (or ILs). Examples of cytokines and chemokines shown to regulate the function of professional antigen presenting cells (APCs) include IL-4 and IL-13, which are known to induce the expression of class II MHC (Major Histocompatability Antigens), activate macrophages and B cells and increase the frequency of Ig class switching (an important process of B cell maturation, which is imperative for the generation of a high affinity humoral response).

Interleukin 4 is a pleiotropic cytokine derived from T cells and mast cells with multiple biological effects on B cells, T cells and many non-lymphoid cells including monocytes, endothelial cells and fibroblasts. It also induces secretion of IgG1 and IgE by mouse B cells and IgG4 and IgE by human B cells. The IL4-dependent production of IgE and possibly IgG1 and IgG4 is due to IL4-induced isotype switching. In humans, IL4 shares this property with IL13.

Interleukin 13 is secreted by activated T cells and inhibits the production of inflammatory cytokines (IL1beta, IL6, TNF alpha, and IL8) by LPS-stimulated monocytes. Human and mouse IL13 induce CD23 expression on human B cells, promote B cell proliferation in combination with anti-Ig or CD40 antibodies, and stimulate secretion of IgM, IgE and IgG4. IL13 has also been shown to prolong survival of human monocytes and increase the surface expression of MHC class II and CD23. Human and mouse IL13 have no known activity on mouse B cells.

Class II MHC are important for the presentation of antigen derived peptides to CD4+ T cells functioning as effector cells in addition to providing support to B cells (secreting high affinity immunoglobulins) and CD8+ T cells (Cytotoxic T Lymphocytes-CTL).

### b. Diketopiperize

Diketopiperize (DKP) acts as a cell-transporter, which facilitates the delivery of associated molecules (e.g. drugs, therapeutics or vaccines) into cells and across tissues.

FDKP microparticles are self-assembling complexes, which are insoluble and stable at one pH and become unstable and/or soluble at another pH. FDKP microparticles are generally about two microns in diameter. In a preferred embodiment, the DKPs are soluble at neutral or physiological pH. FDKP microparticles and methods for making FDKP microparticles are described in U.S. Patent Nos. 5,352,461; 5,503,852; and 6,471,497, incorporated herein by reference. U.S. Patent Nos. 5,877,174; 6,153,613; 5,693,338, 5,976,569; 6,331,318; and 6,395,774 describe substituted and derivatized DKPs and are herein incorporated by reference. FDKP (3,6-Bis [N-Fumaryl-N-(n-butyl)amino]-2,5-DKP, CAS Registry[#] 176738-91-3) has the following structure:

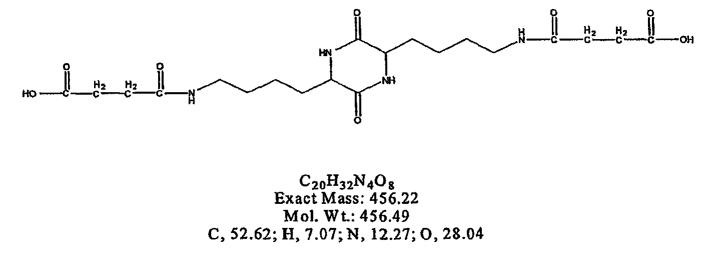

FDKP microparticles are formed by precipitation of DKP droplets into a solution. Compositions such as therapeutic agents (e.g., insulin) were formulated into a stabilized complex by precipitation in an acidic solution with fumaryl DKP. Upon administration to an individual, the DKP microparticles rapidly dissolve, leaving a convoluted, high surface area matrix formed by the natural or synthetic polymer precipitated around the DKP microparticles. By precipitating the DKPs with the agent to be tested, a dense concentration of agent within the matrix is achieved.

The DKPs may be symmetrically functionalized, wherein the two side-chains are identical. Alternatively, the DKPs may be asymmetrically functionalized. Both the symmetrically and asymmetrically functionalized DKPs can have side-chains that contain acidic groups, basic groups, or combinations thereof.

DKPs with zero, one and two protecting groups on the two side-chains each have different solubilities, depending on the solvent and the solution pH, and are isolated from solution by precipitation. Accordingly, selectively deprotecting and precipitating DKPs with one side-chain deprotected yields the unsymmetrical substituted DKPs. The monoprotected DKP derivatives themselves tend to be soluble in acidic media and insoluble in weak alkaline solutions.

TECHNOSPHEREs^{®} is the name given to microparticles formed of DKPs developed by MannKind Corporation (previously known as Pharmaceutical Discovery Corporation). In multiple clinical trials involving frequent pulmonary administrations, TECHNOSPHEREs^{®} exhibited a desired safety profile for delivery of insulin in Type I and Type II diabetic patients.

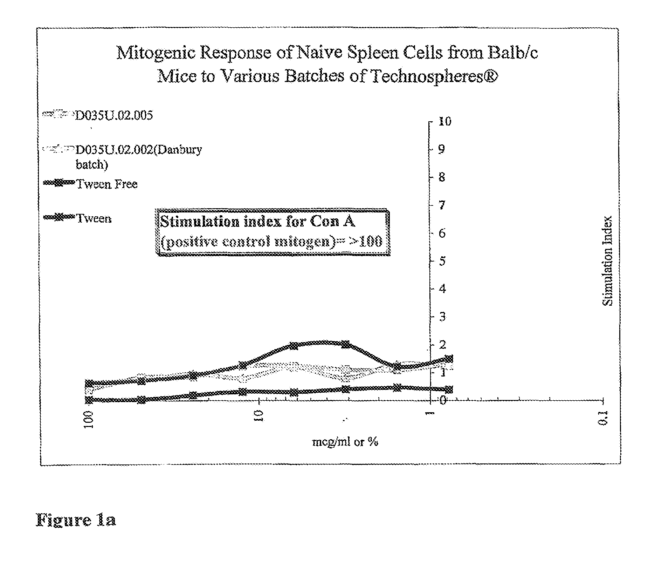

The FDKP microspheres (TECHNOSPHEREs^{®} are inert (see Figures 1a-2b), and enhance cellular uptake without substantial adverse side effects.

FDKP particles expedite the uptake of diverse sets of molecules, including small, organic molecules, biopolymers such as proteins and peptides, and nucleic acids, into cells with retention of biological activity. Both small (e.g., insulin, approximately 5-6 kDa) and larger (e.g., chicken albumin; 45 kDa) proteins are effectively transported into cells.

### c. Size and Weight of Microparticles

To achieve preferential delivery to deep lung tissue, the size of the composition/DKP complex is less than 20 microns in diameter, preferably less than 10 microns, and more preferably less than 5 microns. Particles larger than 5 microns are usually too large to gain access to deep tissues (alveoli) of the lung. For pulmonary delivery, for example, the size is less than 2.5 microns in diameter, e.g., the diameter of the complexes is in the range of 1.5-2.5 microns.

The size/structure of the complex favors efficient transport across cell membranes and minimizes immune stimulation. The molecular weight of the composition is less than 200 kDa, e.g., more preferably less than 100 kDa. Preferably, the molecular weight is less than 50 kDa. More preferably, the molecular weight of the composition is less than 20 kDa or less than 10 kDa (e.g., in the range of 3-6 kDa). For example, a human insulin (molecular weight between 5-6 kDa) is efficiently delivered with substantially no immune stimulation.

### d. Dosage

The dose of composition delivered favors high zone tolerance and/or clonal anergy, thereby ensuring immune nonresponsiveness to the administered compositions. For example, the dose of the composition is in the range of 0.5-100 milligrams per administration. Preferably, the dose of inhaled insulin is in the range of 500-1000 micrograms per administration (typically in the range of 1-4 milligrams per administration or 4-16 milligrams per day) for human administration.

### e. Coatings on DKP

DKP microparticles may be coated with materials such as natural and/or synthetic polymers, most preferably biodegradable polymers. Representative natural polymers include proteins such as albumin, preferably human, fibrin, gelatin, and collagen, and polysaccharides such as alginate, celluloses, dextrans, and chitosans. Representative synthetic polymers include polyhydroxy acids such as polylactic acid (PLA), polyglycolic acid (PGA), copolymers thereof (e.g. poly(lactic-co-glycolic acid ) (PLGA)), polyanhydrides, polyorthoesters, polyhydroxyalkanoates, and although not preferred, non-biodegradable polymers such as polyacrylic acid, polystyrene, and polyethylenevinylacetate..

### II. Methods of Making Compositions

The FDKP microparticles are preferably formed in the presence of a desired compound to be encapsulated by:
(1) Acidification of weak alkaline solutions of a DKP derivative that contains one or more acidic groups,
(2) Basification of acidic solutions of a DKP derivative that contains one or more basic groups, or
(3) Neutralization of an acidic or basic solution of a DKP derivative that contains both acidic and basic groups.

Optionally, the DKP microparticles may be coated with a polymer by precipitating the DKP particles within a matrix of a natural or synthetic polymer.

Modifying the side-chains on the DKP, the concentration of various reactants, the conditions used for formation, and the process used in formation can control the size of the resulting microparticles.

### III. Uses of Compositions

Acceleration and augmentation of transport into target cells following the administration of compound-associated DKPs preparations is one example for the use of this method for improving therapeutic applications.

The DKP complex preparations as microparticles or suspensions (made in phosphate buffered saline at pH 7.4) are administered to target cells such as deep lung tissue. The DKP-compound complexes are administered to a mucosal surface (pulmonary, nasal, vaginal, rectal, or oral) using a schedule and dose which minimizes an immune response. Appropriate concentrations and immunization schedules are determined using standard techniques and are optimized for each compound. Therapeutic compositions (e.g., insulin) are administered in a milligram dose range (thereby avoiding immune stimulation by development of high zone tolerance).

A method of delivering a composition to a specific site in a human or other mammal is carried out by contacting cells or a tissue with a complex containing the compound and DKP. In a preferred embodiment, compositions are delivered to small airways of the lung, e.g., the aveoli. Optionally, the compositions are administered orally, but are not typically administered subcutaneously or intradermally, intravenously, intraperitoneally, or intramuscularly. In one embodiment, the compositions are administered by inhalation.

The method preferably includes a plurality of contacting steps in a defined time period. For example, the interval of time between contacting steps may be less than 24 hours. Complexes may be delivered several times a day. Thus the time period between contacting steps may be less than 12 hours, less than 6 hours, or less than 3 hours. Following a plurality of contacting steps, immune cells in the tissue are nonresponsive to subsequent contact with the composition.

With respect to scheduling, immune cells require a rest period of several days to weeks or months after responding to an initial stimulus before receiving a second stimulation to achieve a potent antigen-specific immune response. When insulin is inhaled, the compositions are typically administered to a patient three or four times a day. This schedule is characterized by a very short interval between stimulations, and thus, does not allow immune cells to become quiescent and receptive for a subsequent signal. The schedule should lead to tolerance, anergy, or apoptosis of antigen-specific immune cells and does not produce a positive immune response.

### Administration of coated DKP microparticles.

In one embodiment, coated diketopiperazines are administered so that a depot forms after the composition is administered to a patient. Following dissolution of the diketopiperazine upon expose to neutral pH, antigen is released and the remaining coating is in the form of a multi-faceted labyrinth-like structure which contains a high local concentration of antigens. The antigens attract peripheral immune cells to the depot, which lead to a high concentration of effector cells, cytokines, and chemokines. The depot provides the necessary components for triggering a vigorous immune response or regulating the immune response to an antigen.

The present invention will be further understood by reference to the following non-limiting examples.

### Examples

### Example 1: Fumaryl DKP does not stimulate innate immunity

To rule out the possibility that DKP possesses immunostimulatory properties due to either its chemical composition or the possible mimicry of pathogenic sequences, e.g., killed *M tuberculosis,* splenocytes from naïve Balb/c mice were incubated with three batches of 'blank' Fumaryl DKP (FDKP) formulated as microparticles and compared with FDKP-associated with OVA ('TCNSP*OVA') at various concentrations. This assay was selected due to the heightened sensitivity of resting T cells to minute quantities of contaminants or mitogens resulting in excitation and proliferation of these cells. Proliferative responses of the splenocytes were measured by a ³H-Thymidine incorporation assay. The FDKP blank microparticles from the various batches induced a comparable proliferation to a control (medium alone). These data indicate that the FDKP is not immunostimulatory.

An analysis of cytokines (IFN_{γ}, TNF-_{α}, IL-4, IL-5 and IL-2) secreted by the cultures was also carried out This assay was used as a second confirmatory assay to examine the mitogenicity of the FDKP microspheres using naive mouse spleen cells cultured for 5 days. Figure 1a is a line graph showing a mitogenic response of naïve spleen cells to Fumaryl DKP (FDKP)-microspheres (TECHNOSPHERE^{®}). The mitogenicity assay was performed using a pool of splenocytes harvested from naïve mice. Naïve cells were plated at 5 x 10⁵ cells/well in a 96 well u-bottom tissue culture treated plate. The cells were incubated with 100 µg/ml of various batches of TECHNOSPHEREs^{®} (including a clinical grade blank TECHNOSPHERE^{®} batch, TWEEN^{®}-free clinical-grade batch and 2 crude batches). TWEEN^{®} 80 (100%) was also included in the test. All samples were titrated 2-fold 7 times to a concentration of 0.7 µg/ml TECHNOSPHERE^{®} or 0.7% TWEEN^{®} 80. To assess the background levels of mitogenicity, cells were incubated with medium alone. To determine the maximum level of stimulation, cells were incubated with Concanavalin A (Con A). Cells were incubated for 72 hours at 37 °C, 5 % CO₂. The cultures were pulsed with 100 µCi/ ml of ³H-thymidine and incubated an additional 16 hours. The percentage of mitogenicity was calculated from the values of ³H-thymidine incorporation that were recorded for the assay as compared with the medium control.

Cytokine analysis was performed using the BD Biosciences Pharmingen Cytometric Bead Array (CBA) Kit for Mouse Th1/Th2 Cytokine Analysis. The supernatant was harvested from cells incubated in the presence of 100 µg/ml of TECHNOSPHERE^{®} associated-Ova (batch numbers 202.24.1, 202.33.1 and 202.040) and in the presence of blank TECHNOSPHEREs^{®} (batch number D-035U.02.002). Levels of IFN-γ, TNF-α, IL-5, IL-4 and IL-2 were quantified using a standard curve for each cytokine.

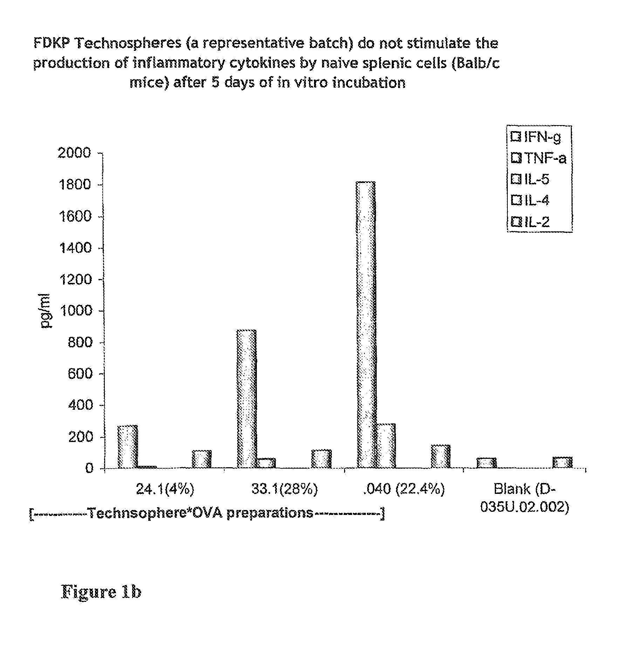

As depicted in Figure 1b, high levels of γIFN, TNF-α, and IL-2 were shown for cultures incubated with Ovalbumin (positive control), whereas insignificant levels of any of the cytokines were recorded for the various batches of FDKP microspheres.

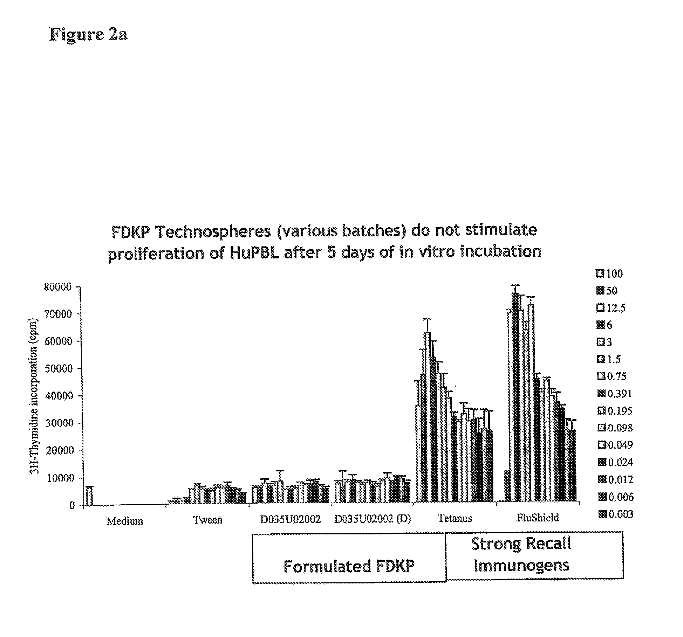

In addition, FDKP was shown to be devoid of mitogens capable of stimulating human peripheral blood lymphocytes (huPBL) in five-day cultures (see Figure 2a). A mitogenicity assay was performed using PBMC's isolated from lymphocyte preps. Naive cells were plated at 5 x 10⁵ cells/well in a 96 well u-bottom tissue culture treated plate. The cells were incubated with 100 microg/ml and subsequent 2-fold serial dilutions of tetanus toxoid or several blank TECHNOSPHERE^{®} batches, including a TWEEN-free clinical-grade batch and several crude (no TWEEN) batches. To assess the background levels of mitogenicity, cells were incubated with medium alone. To determine the maximum level of stimulation, cells were incubated with Phytohemagglutin (PHA). Cells were incubated for 72 hours at 37°C, 5 % CO₂. The cultures were pulsed with 100µCi/ ml of ³H-thymidine and incubated an additional 16 hours. The percentage of mitogenicity was calculated from the values of ³H-thymidine incorporation recorded for the assay as compared with the medium.

Various batches of formulated blank (i.e., unloaded) FDKP TECHNOSPHEREs^{®} (D035U.02.002, D035U.02.002, or TWEEN-free) or crude, unformulated FDKP TECHNOSPHEREs^{®} (001.E.02-011, and 001.E.02-012) did not stimulate huPBL to proliferate above the medium control base line. A strong recall antigen, tetanus toxoid, was used as a positive control to demonstrate an antigen-specific proliferative response (see Figure 2a).

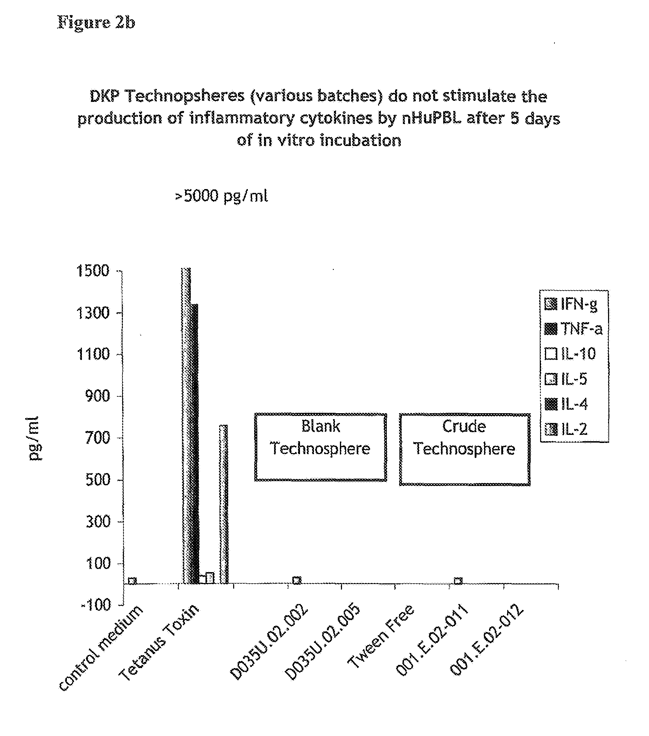

Analysis of cytokines secreted by these cultures was used as a second confirmatory assay to examine the mitogenicity of the.FDKP microspheres using HuPBL. High levels of yIFN, TNF-α, and IL-2 were shown for cultures incubated with tetanus toxoid (positive control) whereas insignificant levels of any of the cytokines were recorded for the various batches of FDKP microspheres (see Figure 2b). Thus, FDKP failed to stimulate an innate immune response, indicating that its mechanism of action is different than the classical bacterial adjuvants or DNA snippets, which are capable to engage toll-like receptors (e.g., TLR-2, 3, 4, 5, or 9).

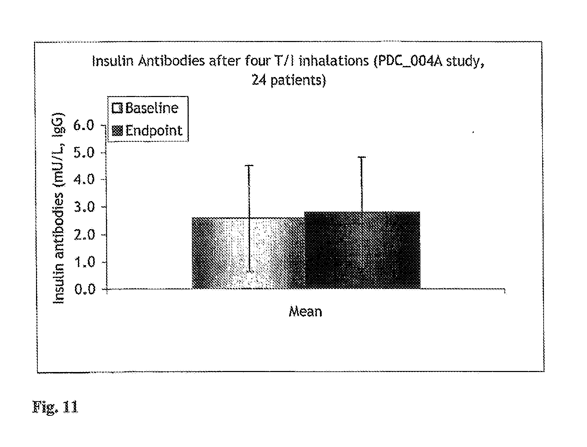

Experiments to evaluate immunogenicity were also carried out in *vivo.* Insulin DKP- microspheres were administered to human subjects by inhalation therapy. 12U, 24U or 48U of insulin doses (corresponding to 450 micrograms, 900 micrograms and 1.8 milligrams of insulin, respectively) formulated with FDKP (particles with a median diameter of 2 microns, and with diameters in the range of 1-5 microns) were administered 6 times in intervals of one week between treatments. Serum samples were obtained from the subjects prior to and after treatment (after six inhalations). Figure 11 is a bar graph showing insulin-specific IgG antibody titers in human subjects before ("baseline") and after ("endpoint") administration of insulin/FDKP-microsphere complexes by inhalation therapy. As depicted in Figure 11, pulmonary administration of insulin-FDKP-microsphere complexes did not result in an increase of insulin-specific antibodies in the sera of treated patients.

### Example 2: Transport kinetics

Uptake experiments were conducted using ovalbumin (OVA) as the transport compound.

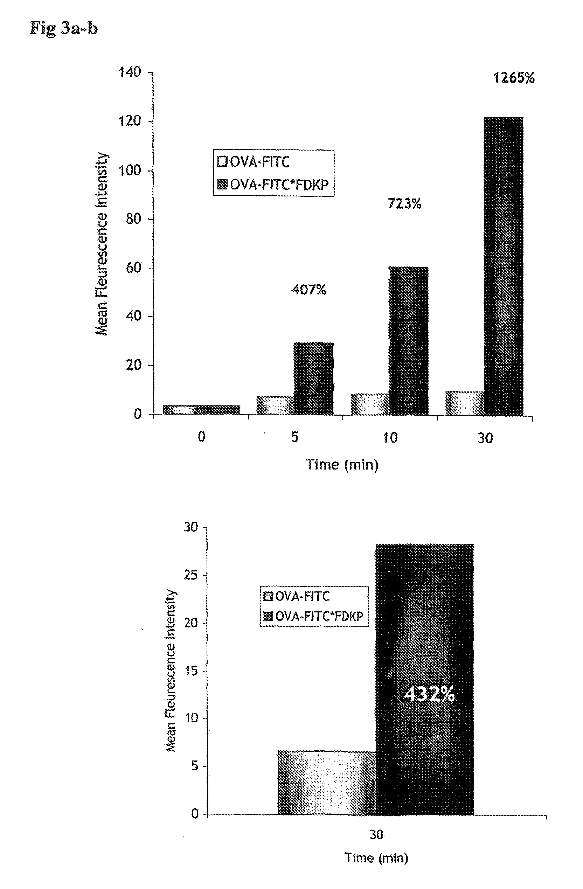

In one experiment, lung cells were incubated with the transport compound at varying incubation times. As shown in Figures 3a and 3b, approximately 50% of transport for OVA was achieved in the first 10 minutes with complete saturation (100%) occurring within 30 minutes at 37°C. These data indicate that uptake of a compound by cells is increased by the presence of FDKP.

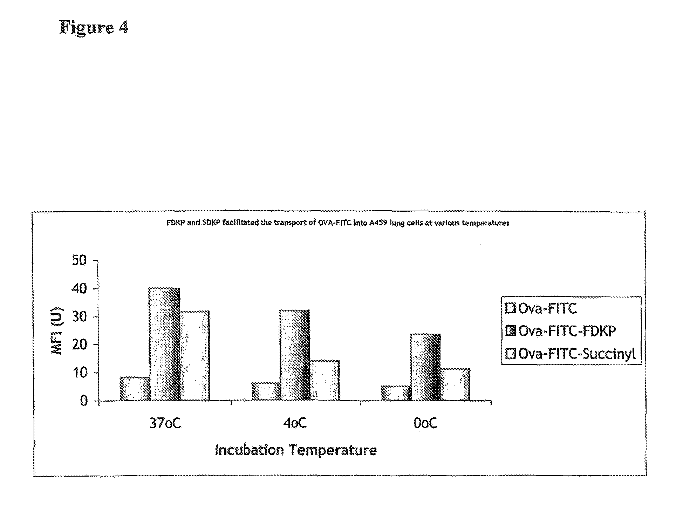

Figure 4 is a bar graph showing transport of OVA-FITC into A459 human lung cells after a 30-minute incubation of 20 micrograms/ml of OVA-FITC-succinyl or OVA-FITC-FDKP (OVA*TECH-FITC) at 37°C, 4°C, and 0°C. Cells were contacted with OVA or OVA-FDKP-microsphere complexes or OVA-Succinyl FDKP- microsphere complexes for 30 minutes prior to measuring fluorescence (as an indication of transport of the compound into the cells). Both complexes had greatly improved transport for all temperatures compared the transport for OVA-FITC without FDKP. OVA-FITC-FDKP had the greatest improvement in transport.

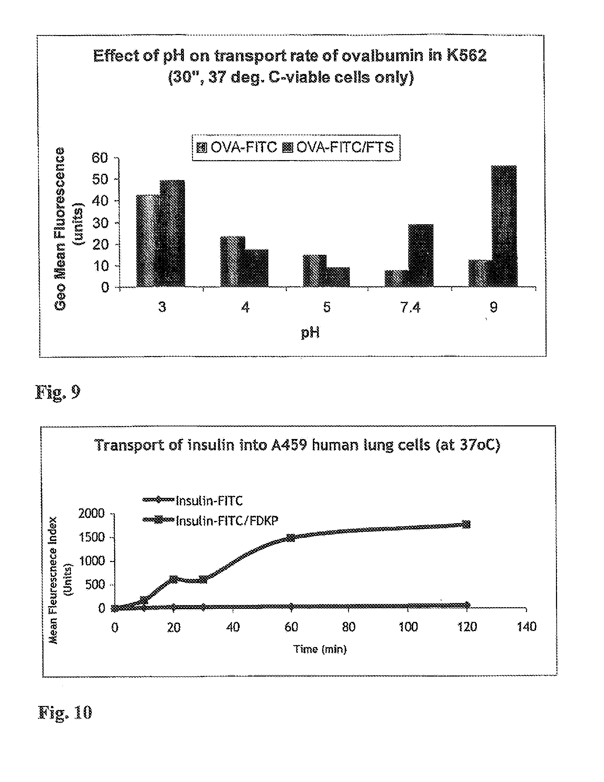

Transport of insulin into lung cells was also evaluated (see Figure 10). Figure 10 is a line graph showing that insulin was not transported into the lung cells, while the insulin/FDKP complex was transported into the lung cells. The data indicate significant cellular uptake in 30-60 minutes and a 28-40 fold enhancement of insulin uptake when associated with DKP-microspheres compared to insulin in the absence of DKP-microspheres.

### Example 3: Transport Enhancement in Spleen Cells

Uncultured primary cells were used to study the rate of transport of a compound into target cells. A time course comparing the rate of transport of the compound using isolated murine spleen cells was performed. Spleens from BALB/C mice were removed, and cell suspensions were prepared. Isolated cells were incubated in complete media (RPMI 1640 +10%FBS, 1X Pen/Strep) at a density of 4 x10⁶ cells/mL. Ovalbumin-FITC or Ovalbumin-FITC/FDKP was added at a concentration of 20 µg/mL, and cells were incubated for indicated times at 37°C. Eight volumes of PBS were added at the end of each incubation period, and cells were kept on ice until the completion of all time points. Cells were centrifuged, re-suspended and analyzed by FACS for FITC uptake.

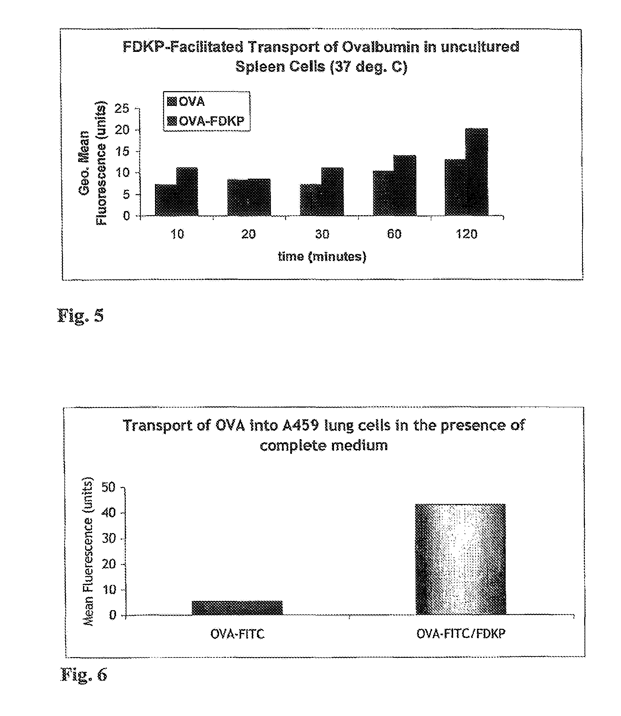

Figure 5 is a bar graph showing FDKP-microsphere-facilitated transport of a test compound, ovalbumin, in uncultured spleen cells at 37° C. Enhancement in the uptake of ovalbumin by spleen cells was witnessed within 10 minutes in the presence of TECHNOSPHERE, demonstrating the rapid and universal enhancement in membrane penetration in cell types studied thus far (see Figure 5). After sixty minutes of incubation with OVA-FITS/FDKP, the presence of another distinct cell population became apparent The viability of cells did not appear to be adversely affected.

### Example 4: Transport in Media Containing Serum

Transport of Ovalbumin-FITC was measured in the presence of serum, a condition relevant to an *in vivo* clinical application. K562 Cells were incubated with either Ovalbumin-FITC or Ovalbumin-FITC/FDKP (30 minutes, 37° C, 20 µg/mL) at a cell density of 4x10⁶ cells/mL in media alone or media w/10% FBS. After washing, cells were analyzed by FACS for FITC incorporation. Prior to analysis, cells were stained with VIAPROBE, a cell viability stain.

Figure 6 is a bar graph showing transport of ovalbumin into A459 lung cells in the presence of complete medium. A more than 5-fold enhancement in intra-cellular ovalbumin content was noted in the presence of serum after 30 minutes at 37° C (see Figure 6).

### Example 5: Transport Enhancement Over A Wide-Range of pH

K562 cells were incubated with either Ovalbumin-FITC or Ovalbumin-FITC/FDKP (30 minutes, 37°C, 20 µg/mL) at a cell density of 4x10⁶ cells/mL in BD cell staining solution (BD Pharmingen) adjusted to pH 3,4, 5,7.4, or 9. After washing, cells were stained 5 minutes on ice with VIAPROBE (BD Pharmingen), and FITC content of viable cells was determined by FACS analysis.

Transport enhancement by TECHNOSPHERE was detected at all pHs studied except pH 4 and 5 (see Figure 9). As depicted in Figure 9, enhancement was particularly significant (nearly a 5-fold increase) at pH 9. These data indicate that FDKP-microspheres are particularly effective at augmenting transport of an associated compound across a cell membrane in various regions of the body characterized by a wide range of pH, including those that are characterized by alkaline conditions, e.g. the intestinal tissue.

### Example 6: Effect of Cross linkers on Transport Enhancement

Studies were carried out to to ensure that DKP microsphere-enhanced transport does not occur by receptor-mediated endocytosis via Clathrin-coated pits, which are noted to be involved in receptor-mediated endocytosis and are responsible for the cellular uptake of certain toxins, lectins, viruses, serum transport proteins, antibodies, hormones, and growth factors. The formation of these pits is inhibited in the presence of a hyperosmolar sucrose solution. Cross-linking of membrane thiol groups is another means of preventing endocytosis, as thiol groups play an important role in membrane transport of a number of molecules, including water, urea, and amino acids. Thiol redox states are also critical in maintaining membrane barrier function. Cross-linking membrane thiol groups with phenylarsine oxide were used to test whether TECHNOSPHEREs are dependent on endocytosis.

K562 cells were pretreated with 80 microM phenylarsine oxide (SIGMA) in serum free RPMI media (5 minutes, 37°C). Cells were washed in PBS twice before incubating cells in 10% serum-containing media with either ovalbumin-FITC or ovalbumin-FITC/FDKP (30 minutes, 37°C, at cell and label conditions indicated for previous transport studies). For the effect of a hyperosmolor sucrose solution, the incubations were carried out in the presence of media containing 0.5M sucrose. Cells were washed and analyzed by FACS. Viability of cells after treatment was assessed with VIAPROBE, as indicated previously, and analysis reflects viable cells only.

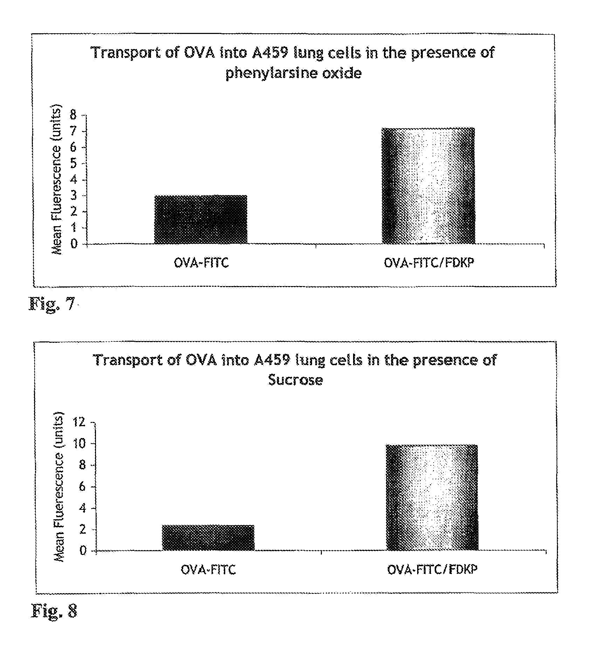

Figure 7 is a bar graph showing transport of ovalbumin into A459 lung cells in the presence of phenylarsine oxide. Figure 8 is a bar graph showing transport of ovalbumin into A459 lung cells in the presence of sucrose. With both treatments, TECHNOSPHERE-mediated transport enhancement was diminished relative to enhancement seen in complete media alone. Enhancement was still observed, however, indicating that TECHNOSPHERE's mechanism of transport enhancement is still effective despite the significant alterations to the membrane by these treatments (Figures 7 and 8).

### Example 7: DKP analogs as transporters to facilitate drug delivery

A succinyl analog of DKP was evaluated as a facilitator of intracellular transport of compound. The succinyl analog of DKP was allowed to associate with OVA, and human lungs cells were contacted with the complexes. Figure 4 depicts the enhanced transport of OVA associated with succinyl DKP and fumaryl DKP as compared to OVA alone.

### Example 8: Tissue-targeted delivery of DKP complexes fails to stimulate an immune response

Dose of composition, administration schedule, size/structure of composition, and site of administration were optimized to achieve efficient drug delivery to cells with minimal or no stimulation of the immune system. For example, delivery of insulin by inhalation to achieve deep lung deposition (using small, e.g., 2 micron insulin/FDKP complexes, which are deposited in alveoli of the lungs) at a dose range of 1-20 mg/kg/day did not stimulate an immune response. The deep lungs (alveoli) provide for an environment that does not support the development of immune response, thereby avoiding development of a deleterious response to inhaled small particles. Micron-sized small particles can gain access to deep respiratory tissues (e.g., alveoli), and environments characterized by immune suppressing conditions. In contrast, larger particles (>5-10 microns) are deposited in the upper respiratory tract Such larger particles do not gain access to immune suppressing conditions of the alveolar tissue, and therefore, may stimulate an immune response.

The dose of insulin given in one treatment far exceeds the amount of peptide used to elicit an immune response. Rather then inducing an immune response, the administered dose induces immune non-responsiveness (e.g., tolerance, clonal anergy). For example, peptide administered in the microgram dose range (e.g., 50 microg) (example i.m. vaccine) stimulates an immune response, whereas 5 mgs or 10 mgs of Insulin/FDKP complexes given by inhalation is expected to not result in stimulation of an immune response.

### Example 9: Effect of Size of Compound to be Delivered

The structure and the size of the compound to be delivered also have an impact on its immunogenicity. Small peptides are less immunogenic, while large heterogeneous or complex molecules are more immunogenic. The human insulin composition tested (molecular weight of 5807.6 Daltons) is anticipated to have a lower probability of stimulation an immune response when delivered to pulmonary tissue. To further minimize immune stimulation, an immune compatible composition is used. For example, a human form of insulin is a weakly immunogenic antigen in humans.

A clinical study was conducted with 24 patients to evaluate immune responsiveness. Patients were treated 4 times with insulin/DKP complexes (molecular weight of insulin 5806, complex size of approximately 2 microns) by inhalation. The level of anti-insulin antibodies detected after treatment was not different from the pre-treatment level, as measured by IgG ELISA (Figure 11). These data indicate that the drug delivery compositions and methods described herein do not stimulate a clinically relevant immune response.

### The following represent possible embodiments according to the invention:

1. A method for transporting a compound across a membrane or lipid bilayer, comprising contacting a proximal face of the membrane or bilayer with a complex comprising the compound and diketopiperazine (DKP), wherein transport of the compound from the proximal face of the lipid bilayer to a distal face of the lipid bilayer is increased in the presence of the DKP compared to in the absence of the DKP.
2. The method of embodiment 1, wherein the lipid bilayer comprises an intact cell.
3. The method of embodiment 2, wherein substantially no immune response is induced following contact of the cell with the complex.
4. The method of embodiment 3, wherein the immune response is increased by less than 20% in the presence of the DKP compared to in its absence.
5. The method of embodiment 1, wherein the compound is a biologically active agent.
6. The method of embodiment 5, wherein the biologically active agent is selected from the group consisting of insulin, an insulin precursor, Parathyroid hormone (PTH), Calcitonin, Human Growth Hormone (HgH), Glucagon-like peptides (GLP), cytokines, chemokines, and fragments thereof.
7. The method of embodiment 5, wherein the biologically active agent is an antibody or fragment thereof.
8. The method of embodiment 1, wherein the diameter of the complex is less than 5 microns.
9. The method of embodiment 1, wherein the diameter of complex is less than 2.5 microns.
10. The method of embodiment 1, wherein the diameter of the complex is between 1.5 and 2.5 microns.
11. The method of embodiment 3, wherein the immune response is measured by detecting an antibody, T cell proliferation, or production of a cytokine.
12. The method of embodiment 11, wherein the cytokine is interleukin-2.
13. The method of embodiment 1, wherein DKP does not engage a toll-like receptor.
14. The method of embodiment 1, wherein a pulmonary tissue or cells are contacted.
15. The method of embodiment 14, wherein the pulmonary tissue comprises a small airway of the lung.
16. The method of embodiment 14, wherein the tissue comprises alveoli.
17. The method of embodiment 14, wherein a dose of the compound is between 0.5 and 100 milligrams per administration.
18. The method of embodiment 14, wherein a dose of the compound is between 500 and 1000 micrograms per administration.
19. The method of embodiment 14, wherein a dose of the compound is between 2 and 16 milligrams per day.
20. The method of embodiment 14, wherein the molecular weight of the compound is less than 200 kDa.
21. The method of embodiment 14, wherein the molecular weight of the compound is less than 100 kDa.
22. The method of embodiment 14, wherein the molecular weight of the compound is less than 100 kDa.
23. The method of embodiment 14, wherein the molecular weight of the compound is between 3 and 6 kDa.
24. The method of embodiment 14, wherein the composition is a polypeptide.
25. The method of embodiment 24, wherein the amino acid sequence of the polypeptide is identical to a naturally-occurring polypeptide expressed by a member of the species of the mammal.
26. The method of embodiment 24, wherein the polypeptide is an insulin, an insulin precursor, Parathyroid hormone (PTH), Calcitonin, Human Growth Hormone (HgH), Glucagon-like peptides (GLP), or a fragment thereof.
27. The method of embodiment 24, wherein the polypeptide is an antibody or fragment thereof.
28. The method of embodiment 14, wherein the method comprises a plurality of contacting steps.
29. The method of embodiment 28, wherein an interval of time between the contacting steps is less than 24 hours.
30. The method of embodiment 29, wherein the interval is less than 12 hours.
31. The method of embodiment 29, wherein the interval is less than 6 hours.
32. The method of embodiment 29, wherein the interval is less than 3 hours.
33. The method of embodiment 28, wherein following the plurality of contacting steps, immune cells in the pulmonary tissue are non-responsive to subsequent contact with the compound.
34. The method of embodiment 1, wherein the membrane or lipid bilayer is located in a mammal.
35. The method of embodiment 34, wherein the mammal is a human.
36. The method of embodiment 34, wherein the complex is administered orally.
37. A composition for use in the method of any of embodiments 1-37.

## Claims

1. A composition for delivering a compound to a mammal, comprising microparticles of a complex of a diketopiperazine and a Glucagon like peptide (GLP).

2. The composition of claim 1, wherein the complex comprises diketopiperazine microparticles having a coating comprising a Glucagon like peptide.

3. The composition of claim 1 or 2, further comprising a biodegradable polymer.

4. The composition of claim 1 or 2, further comprising a biodegradable polymer coating.

5. The composition of any one of claims 1-4, wherein at least a portion of the microparticles have a diameter of less than 20 microns.

6. The composition of any one of claims 1-4, wherein at least a portion of the microparticles have a diameter of less than 10 microns.

7. The composition of any one of claims 1-4, wherein at least a portion of the microparticles have a diameter of less than 5 microns.

8. The composition of any one of claims 1-4, wherein at least a portion of the microparticles have a diameter of about 1.5 microns to about 2.5 microns.

9. The composition of any one of the preceding claims, herein the composition is substantially non-immunogenic to a host.

10. The composition of any one of the preceding claims, wherein the composition is for use at a mucosal surface of the lungs, nasal, vaginal, rectal or oral cavities.

11. The composition of claim 10, wherein the composition is for use at a mucosal surface of the lungs.

12. A use of a compound according to any one of claims 1-11 for reducing immunogenicity of a Glucagon-like peptide.
